(19) Europäisches Patentamt
European Patent Office
Office européen des brevets

(11) EP 3 360 467 A1

(12) EUROPEAN PATENT APPLICATION

(43) Date of publication:
15.08.2018 Bulletin 2018/33

(51) Int Cl.:
A61B 5/00 (2006.01) A61B 5/145 (2006.01)

(21) Application number: 18000110.9

(22) Date of filing: 07.02.2018

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD TN

(30) Priority: 10.02.2017 JP 2017023538

(71) Applicant: CANON KABUSHIKI KAISHA
Tokyo (JP)

(72) Inventor: ABE, Naoto
Tokyo (JP)

(74) Representative: WESER & Kollegen
Radeckestraße 43
81245 München (DE)

(54) OBJECT INFORMATION ACQUIRING APPARATUS AND DISPLAY METHOD

(57) An object information acquiring apparatus is provided, which includes: a light irradiator emitting with light at first and with light at second wavelength; a receiving unit receiving an acoustic wave and outputs a signal; a processing unit generating structural information and functional information; and a display controlling unit, wherein a first irradiation at the first wavelength, a second irradiation at the second wavelength, and a third irradiation at the first wavelength are performed, the receiving unit outputs first to third signals, the processing unit generates first functional information based on the first and second signals and generates second functional information based on the second and third signals.

FIG. 4

EP 3 360 467 A1

**Description**

BACKGROUND OF THE INVENTION

Field of the Invention

The present invention relates to an object information acquiring apparatus and a display method.

Description of the Related Art

Research and development are underway on photoacoustic apparatuses which image the inside of an object using light. A photoacoustic apparatus performs reconstruction using an acoustic wave (a photoacoustic wave) generated by a photoacoustic effect from a light absorber having absorbed energy of light irradiated on an object and forms an absorption coefficient distribution image. Furthermore, the photoacoustic apparatus generates a structural image or a functional image of the inside of the object from the absorption coefficient distribution image. An example of a structural image is an image indicating a position of a blood vessel inside the object. An example of a functional image is an image indicating a characteristic information distribution corresponding to optical characteristics inside the object. As a functional image, an oxygen saturation distribution image which is acquired using light at a plurality of wavelengths is particularly attracting attention.

In addition, photoacoustic apparatuses capable of readily accessing an observation site using a handheld probe similar to that used in ultrasonic diagnostic apparatuses are being researched and developed. Research and development are underway in order to enable real-time observation of a structural image and a functional image of the inside of an object with such a photoacoustic apparatus including a handheld probe.

Japanese Patent Application Laid-open No. 2016-013421 discloses a method of obtaining, with a photoacoustic apparatus using light pulses which have a plurality of wavelengths and which are emitted at different time points, a functional image (an oxygen saturation distribution image) by correcting motion between light emissions. In addition, Japanese Patent Application Laid-open No. 2015-142740 discloses a method of obtaining a structural image (an image specifying a blood vessel position) using an absorption coefficient distribution image of a first wavelength at which oxyhemoglobin and deoxyhemoglobin have the same absorption coefficient among a plurality of wavelengths.

Patent Literature 1: Japanese Patent Application Laid-open No. 2016-013421
Patent Literature 2: Japanese Patent Application Laid-open No. 2015-142740

SUMMARY OF THE INVENTION

Conventionally, a photoacoustic apparatus adopting a mechanical scanning system is known in which a probe provided on a stage is mechanically scanned to form an absorption coefficient distribution image corresponding to a plurality of light pulses. When the techniques described in Japanese Patent Application Laid-open No. 2016-013421 and Japanese Patent Application Laid-open No. 2015-142740 are applied to such a photoacoustic apparatus adopting a mechanical scanning system, a preferable image is obtained. However, with the techniques described in Japanese Patent Application Laid-open No. 2016-013421 and Japanese Patent Application Laid-open No. 2015-142740, a display image is obtained after light emission at a plurality of wavelengths is finished. Therefore, when observing a structural image or a functional image of the inside of an object in real time, there is a problem in that the number of times a display image is updated is smaller than the number of light emissions. In other words, when displaying a structural image or a functional image in real time, there is a problem in that time trackability of image display in response to a motion of a probe or a body motion declines. In particular, since there is a strong demand for real-time display with respect to photoacoustic apparatuses including a handheld probe, an improvement in time trackability is required. In addition, better time trackability of image display is also desirable for mechanical scanning systems.

The present invention has been made in consideration of the problems described above. The present invention is provided to favorably perform real-time display of an image of the inside of an object with a photoacoustic apparatus.

The present invention in its first aspect provides object information acquiring apparatus as specified in claims 1 to 11.

The present invention in its second aspect provides an object information acquiring apparatus as specified in claims 12 to 14.

The present invention in its third aspect provides display method as specified in claims 15 to 21.

According to the present invention, real-time display of an image of the inside of an object can be favorably performed with a photoacoustic apparatus.

Further features of the present invention will become apparent from the following description of exemplary embodiments with reference to the attached drawings.

BRIEF DESCRIPTION OF THE DRAWINGS

FIG. 1 is a block diagram of a photoacoustic apparatus according to a first embodiment;
FIG. 2 is a schematic diagram of a handheld probe according to the first embodiment;
FIG. 3 is a block diagram showing a computer and a peripheral configuration thereof according to the first embodiment;
FIG. 4 is a timing chart for explaining operations in the first embodiment;
FIGS. 5A and 5B are flow charts for explaining operations in the first embodiment of the present invention;
FIG. 6 is a graph showing examples of absorption coefficients at a first wavelength $\lambda 1$ and a second wavelength $\lambda 2$;
FIG. 7 is a diagram for explaining correction for lowering a threshold; and
FIG. 8 is a timing chart of a "fidelity mode".

DESCRIPTION OF THE EMBODIMENTS

Hereinafter, preferred embodiments of the present invention will be described with reference to the drawings. However, it is to be understood that dimensions, materials, shapes, relative arrangements, and the like of components described below are intended to be modified as deemed appropriate in accordance with configurations and various conditions of apparatuses to which the present invention is to be applied. Therefore, the scope of the present invention is not intended to be limited to the embodiments described below.

The present invention relates to a technique for detecting an acoustic wave propagating from an object and generating and acquiring characteristic information on the inside of the object. Accordingly, the present invention can be considered an object information acquiring apparatus or a control method thereof, or an object information acquiring method and a signal processing method. The present invention can also be considered a display method for generating and displaying an image indicating characteristic information on the inside of an object. The present invention can also be considered a program that causes an information processing apparatus including hardware resources such as a CPU and a memory to execute these methods or a computer-readable non-transitory storage medium storing the program.

The object information acquiring apparatus according to the present invention includes a photoacoustic imaging apparatus utilizing a photoacoustic effect in which an acoustic wave generated inside an object when irradiating the object with light (an electromagnetic wave) is received and characteristic information on the object is acquired as image data. In this case, characteristic information refers to information on a characteristic value corresponding to each of a plurality of positions inside the object which is generated using a signal derived from a received photoacoustic wave.

Photoacoustic image data according to the present invention is a concept encompassing all image data derived from a photoacoustic wave generated by light irradiation. For example, photoacoustic image data is image data representing a spatial distribution of at least one type of object information such as generation sound pressure (initial sound pressure), energy absorption density, an absorption coefficient of a photoacoustic wave, and a concentration of a substance (for example, oxygen saturation) constituting the object. Moreover, photoacoustic image data indicating spectral information such as a concentration of a substance constituting the object is obtained based on a photoacoustic wave generated by irradiating light at a plurality of wavelengths that differ from each other. Photoacoustic image data indicating spectral information may be oxygen saturation, a value obtained by weighting oxygen saturation with intensity of an absorption coefficient or the like, total hemoglobin concentration, oxyhemoglobin concentration, or deoxyhemoglobin concentration. Alternatively, photoacoustic image indicating spectral information may be glucose concentration, collagen concentration, melanin concentration, or a volume fraction of fat or water.

A two-dimensional or three-dimensional characteristic information distribution is obtained based on characteristic information at each position in the object. Distribution data may be generated as image data. Characteristic information may be obtained as distribution information on respective positions inside the object instead of as numerical data. In other words, distribution information such as an initial sound pressure distribution, an energy absorption density distribution, an absorption coefficient distribution, and an oxygen saturation distribution may be obtained.

An acoustic wave according to the present invention is typically an ultrasonic wave and includes an elastic wave which is also referred to as a sonic wave or an acoustic wave. An electrical signal converted from an acoustic wave by a transducer or the like is also referred to as an acoustic signal. However, descriptions of an ultrasonic wave and an acoustic wave in the present specification are not intended to limit a wavelength of such elastic waves. An acoustic wave generated by a photoacoustic effect is referred to as a photoacoustic wave or an optical ultrasonic wave. An electrical signal derived from a photoacoustic wave is also referred to as a photoacoustic signal. Distribution data is also referred to as photoacoustic image data or reconstructed image data.

In the following embodiments, a photoacoustic apparatus which irradiates an object with pulsed light in a plurality of wavelengths that differ from each other, which receives a photoacoustic wave from the object, and which generates a vascular image (a structural image) or an oxygen saturation distribution image (a functional image) of the inside of the object will be discussed as an object information acquiring apparatus. In addition, while a photoacoustic apparatus including a handheld probe is discussed in the following embodiments, the present invention can also be applied to a photoacoustic apparatus which provides a probe on a stage and which performs scanning in a mechanical manner.

First Embodiment

Apparatus configuration

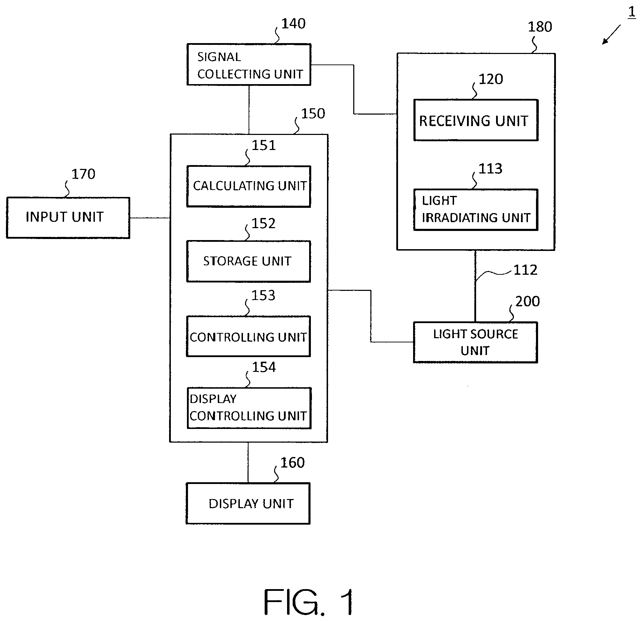

Hereafter, a configuration of a photoacoustic apparatus 1 according to the present embodiment will be described with reference to the schematic block diagram shown in FIG. 1. The photoacoustic apparatus 1 includes a probe 180, a signal collecting unit 140, a computer 150, a display unit 160, an input unit 170, and a light source unit 200. The probe 180 includes a light irradiating unit 113 and a receiving unit 120. The computer 150 includes a calculating unit 151, a storage unit 152, a controlling unit 153, and a display controlling unit 154.

The light source unit 200 supplies the light irradiating unit 113 with a light pulse via an optical system 112 constituted by an optical fiber (a bundle fiber) or the like. The light irradiating unit 113 irradiates an object 100 with the supplied light. The receiving unit 120 receives a photoacoustic wave generated from the object 100 and outputs an electrical signal (a photoacoustic signal) as an analog signal. The signal collecting unit 140 converts the analog signal output from the receiving unit 120 into a digital signal and outputs the digital signal to the computer 150.

The computer 150 stores the digital signal output from the signal collecting unit 140 in a memory as an electrical signal (a photoacoustic signal) derived from a photoacoustic wave. The computer 150 generates photoacoustic image data by performing processing such as image reconstruction on the stored digital signal. In addition, after performing image processing for display on the obtained photoacoustic image data, the computer 150 outputs the image data to the display unit 160. Furthermore, the computer 150 controls the entire photoacoustic apparatus 1.

The display unit 160 displays a photoacoustic image based on photoacoustic image data. A user (a physician, a technician, or the like) can carry out a diagnosis by checking the photoacoustic image displayed on the display unit 160. The display image may be stored in a memory inside the computer 150, a data management system connected to the photoacoustic apparatus via a network, or the like based on a storage instruction from the user or the computer 150. The input unit 170 accepts instructions and the like from the user.

Detailed Configuration of Each Block

Next, a favorable configuration of each block will be described in detail.

Probe 180

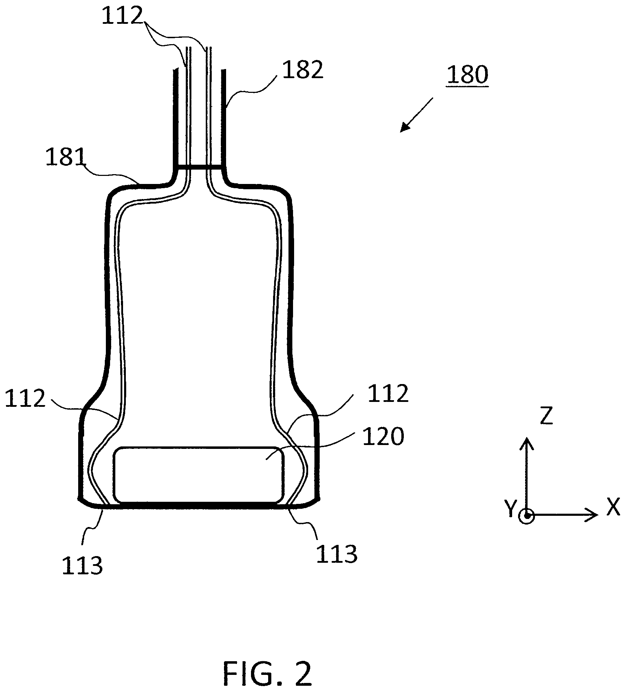

FIG. 2 is a schematic diagram of the probe 180 according to the present embodiment. The probe 180 includes the light irradiating unit 113, the receiving unit 120, and a housing 181. The housing 181 is a housing that encloses the light irradiating unit 113 and the receiving unit 120. By gripping the housing 181, the user can use the probe 180 as a handheld probe. The light irradiating unit 113 irradiates an object with a light pulse propagated by the optical system 112. Moreover, XYZ axes in the diagram represent coordinate axes when the probe is stationary and are not intended to limit orientations when the probe is in use.

The probe 180 shown in FIG. 2 is connected to the signal collecting unit 140 and the light source unit 200 via a cable 182. The cable 182 includes the optical system 112 which supplies a light pulse from the light source unit 200 to the light irradiating unit 113 and wiring (not shown) which outputs an analog signal output from the receiving unit 120 to the signal collecting unit 140. Alternatively, the cable 182 may be provided with a connector and configured so as to enable the probe 180 to be separated from other components of the photoacoustic apparatus.

Optical System 112

The optical system 112 is an optical member which transmits light generated by the light source unit 200 to the light irradiating unit 113. The use of a bundle fiber is recommended from the perspective of attaining preferable handleability. Alternatively, any member for transmitting light such as a prism and a mirror can be used.

Light Irradiating Unit 113

The light irradiating unit 113 is an exit end for irradiating the object with light. A terminal end of a bundle fiber can be used as the light irradiating unit 113. In addition, when a part (such as a breast) of a living organism is used as the object 100, a diffuser plate or the like for disseminating light may be used in order to irradiate the object 100 with pulsed light having a widened beam diameter.

Receiving Unit 120

The receiving unit 120 includes a transducer which receives an acoustic wave and outputs an electrical signal and a supporter which supports the transducer. A transducer can be used which uses, for example, piezoelectric materials, capacitive transducers (capacitive micro-machined ultrasonic transducers: CMUTs), and Fabry-Perot interferometers as a member constituting the transducer. Examples of piezoelectric materials include a piezoelectric ceramic material such as lead zirconate titanate (PZT) and a polymer piezoelectric film material such as polyvinylidene fluoride (PVDF).

An electrical signal obtained by the transducer is a time-resolved signal. Therefore, an amplitude of the electrical signal represents a value based on sound pressure (for example, a value proportional to sound pressure) received by the transducer at each time point. Moreover, favorably, the transducer is capable of detecting a frequency component (typically, 100 KHz to 100 MHz) constituting a photoacoustic wave. Alternatively, arranging a plurality of transducers side by side on the supporter to form a flat surface or a curved surface which is referred to as a 1D array, a 1.5D array, a 1.75D array, or a 2D array is also favorable.

The receiving unit 120 may include an amplifier for amplifying a time-sequential analog signal output from the transducer. In addition, the receiving unit 120 may include an A/D converter for converting a time-sequential analog signal output from the transducer into a time-sequential digital signal. In other words, the receiving unit 120 may include the signal collecting unit 140.

Moreover, in order to improve image accuracy by detecting an acoustic wave from various angles, a transducer arrangement in which the object 100 is surrounded from an entire circumference thereof is favorable. In addition, when the object 100 is too large to be surrounded from an entire circumference thereof, transducers may be arranged on a hemispherical supporter. The probe 180 including the receiving unit 120 shaped in this manner is suitable for a mechanical scanning photoacoustic apparatus in which the probe is moved relative to the object 100 instead of a handheld probe. A scanning unit such as an XY stage may be used to move the probe. Moreover, the arrangement and the number of transducers as well as the shape of the supporter are not limited to those described above and may be optimized in accordance with the object 100.

A medium that enables a photoacoustic wave to propagate is favorably arranged in a space between the receiving unit 120 and the object 100. Accordingly, acoustic impedances are matched at an interface between the object 100 and the transducer. Examples of such a medium include water, oil, and an ultrasonic gel.

The photoacoustic apparatus 1 may include a holding member which holds the object 100 to stabilize a shape of the object 100. A holding member with both high light transmittivity and high acoustic wave transmittivity is favorable. For example, polymethylpentene, polyethylene terephthalate, acrylic, and the like can be used.

When the apparatus according to the present embodiment generates an ultrasonic image in addition to a photoacoustic image by transmitting and receiving acoustic waves, the transducer may function as a transmitting unit that transmits an acoustic wave. A transducer as a receiving unit and a transducer as a transmitting unit may be a single (common) transducer or may be separate components.

Light Source Unit 200

The light source unit 200 is an apparatus that generates light for irradiating the object 100. As the light source unit 200, a variable-wavelength solid-state laser apparatus is preferable in order to generate large-output pulsed light and, at the same time, acquire substance concentrations such as oxygen saturation. Alternatively, a semiconductor laser apparatus or a light source apparatus (for example, a light-emitting diode or a flash lamp) other than a laser may be used. In addition, in order to achieve wavelength variability, a plurality of light source apparatuses which respectively generate light at a different wavelength may be used in combination.

A pulse width of light emitted by the light source unit 200 is, for example, at least 1 ns and not more than 100 ns. In addition, while a wavelength of the light is preferably at least 400 nm and not more than 1600 nm, the wavelength may be determined in accordance with light absorption characteristics of a light absorber to be imaged. When imaging a blood vessel at high resolution, a wavelength (at least 400 nm and not more than 700 nm) which is well absorbed by the blood vessel may be used. When imaging a deep part of a living organism, light at a wavelength (at least 700 nm and not more than 1100 nm) which is only weakly absorbed by background tissue (water, fat, and the like) of the living organism may be used.

In the present embodiment, titanium-sapphire laser (Ti:S) is used as the light source unit 200. Nd:YAG laser light (nanosecond-order pulsed light at a wavelength of 1064 nm) is used to excite Ti:S. The light source unit 200 emits light at two wavelengths of at least 700 nm which are capable of reaching deep parts of the object. A first wavelength λ1 is 797 nm. At the first wavelength, absorption coefficients of oxyhemoglobin and deoxyhemoglobin are substantially equal to each other. Moreover, when selecting a wavelength, a wavelength at which a structural image is preferably displayed may be used. For example, using a wavelength of at least 778 nm and not more than 950 nm enables, when subjecting a reconstructed absorption coefficient distribution image to a binarization process and trimming using a 30-percent value of a maximum absorption coefficient in the image as a threshold, a variation in the size of a blood vessel to be kept within ±10%. In addition, the terms "first wavelength" and "second wavelength" are used simply for the sake of convenience and any one of two wavelengths may be referred to as the first wavelength.

A second wavelength λ2 is 756 nm. Since the absorption coefficient of deoxyhemoglobin peaks at this wavelength, there is a large difference between the absorption coefficients of oxyhemoglobin and deoxyhemoglobin. When calculating oxygen saturation, selecting a wavelength at which a difference in the absorption coefficients of the two types of hemoglobin is large and which has an absorption coefficient comparable to the absorption coefficient with respect to light at the first wavelength of 797 nm as is the case of the second wavelength of 756 nm enables oxygen saturation to be accurately acquired.

Signal Collecting Unit 140

The signal collecting unit 140 includes an amplifier which amplifies an electrical signal that is an analog signal output from the receiving unit 120 and an A/D converter which converts an analog signal output from the amplifier into a digital signal. The signal collecting unit 140 may be constituted by a field programmable gate array (FPGA) chip or the like. A digital signal output from the signal collecting unit 140 is stored in a storage unit 152 inside the computer 150. The signal collecting unit 140 is also referred to as a data acquisition system (DAS). In the present specification, an electrical signal is a concept encompassing both analog signals and digital signals. Moreover, by connecting a light detecting sensor which detects light from the light source unit 200 to the signal collecting unit 140, light irradiation and processing of signal collection can be synchronized.

As described earlier, the signal collecting unit 140 may be arranged inside the housing 181 of the probe 180. With such a configuration, since information between the probe 180 and the computer 150 is to be propagated using digital signals, noise immunity is improved. In addition, the use of high-speed digital signals enables the number of wirings to be reduced and operability of the probe 180 to be improved as compared to transmitting analog signals.

Computer 150

The computer 150 includes the calculating unit 151, the storage unit 152, the controlling unit 153, and the display controlling unit 154. A unit which provides a calculation function as the calculating unit 151 may be constituted by a processor such as a CPU or a graphics processing unit (GPU) or an arithmetic circuit such as a field programmable gate array (FPGA) chip. Such units may be constituted by a single processor or a single arithmetic circuit or may be constituted by a plurality of processors or a plurality of arithmetic circuits. The calculating unit 151 generates photoacoustic image data (a structural image or a functional image) by image reconstruction and executes other kinds of arithmetic processing. The calculating unit 151 may accept from the input unit 170 input of various parameters including object sound velocity and a configuration of a holding unit and may use the parameters in calculations.

As a reconstruction algorithm used by the calculating unit 151 to convert an electrical signal into three-dimensional volume data, any method such as a time-domain back-projection method, a Fourier domain back-projection method, and a model-based method (a repeat operation method) can be adopted. Examples of a time-domain back-projection method include universal back-projection (UBP), filtered back-projection (FBP), and phasing addition (Delay-and-Sum).

When using two wavelengths, the calculating unit 151 generates, by an image reconstruction process, a first initial sound pressure distribution from a photoacoustic signal derived from the light at the first wavelength and a second initial sound pressure distribution from a photoacoustic signal derived from the light at the second wavelength. In addition, a first absorption coefficient distribution is acquired by correcting the first initial sound pressure distribution with a light amount distribution of the light at the first wavelength and a second absorption coefficient distribution is acquired by correcting the second initial sound pressure distribution with a light amount distribution of the light at the second wavelength. Furthermore, an oxygen saturation distribution is acquired from the first and second absorption coefficient distributions. Moreover, since all that is needed is that an oxygen saturation distribution be eventually obtained, contents and a sequence of calculations are not limited to the above.

The storage unit 152 can be constituted by a non-transitory storage medium such as a read only memory (ROM), a magnetic disk, and a flash memory. Alternatively, the storage unit 152 may be a volatile medium such as a random access memory (RAM). Moreover, a storage medium in which a program is to be stored is a non-transitory storage medium. In addition, the storage unit 152 is not limited to a configuration having a single storage medium and may be constituted by a plurality of storage media.

The storage unit 152 is capable of storing various types of data including photoacoustic image data generated by the calculating unit 151 and a display image based on the photoacoustic image data.

The controlling unit 153 is constituted by an arithmetic element such as a CPU. The controlling unit 153 controls operations of each component of the photoacoustic apparatus. The controlling unit 153 may cohtrol operations of each component of the photoacoustic apparatus upon receiving instruction signals in accordance with various operations such as start of measurement from the input unit 170. In addition, the controlling unit 153 controls operations of each component of the photoacoustic apparatus by reading a program code stored in the storage unit 152.

The display controlling unit 154 shares a common configuration with or has a similar configuration to the controlling unit 153. The display controlling unit 154 outputs image data to the display unit 160 and performs image adjustment. Accordingly, oxygen saturation distribution images are sequentially displayed in accordance with probe movement and photoacoustic measurement.

The computer 150 may be a work station exclusively designed for the present invention. Alternatively, the computer 150 may be a general-purpose PC or work station which is operated according to instructions of a program stored in the storage unit 152. In addition, each component of the computer 150 may be constituted by a different piece of hardware. Alternatively, at least a part of the components of the computer 150 may be constituted by a single piece of hardware.

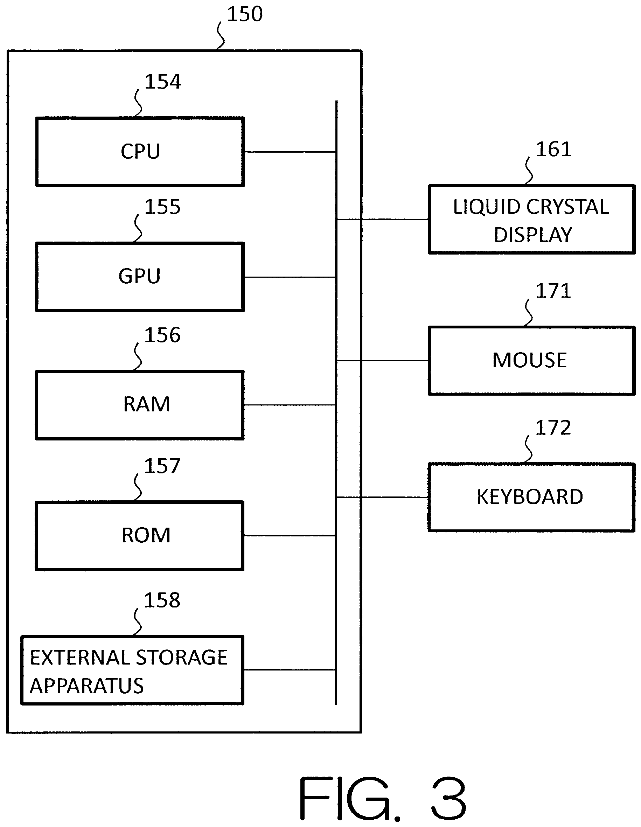

FIG. 3 shows a specific configuration example of the computer 150 according to the present embodiment. The computer 150 according to the present embodiment is constituted by a CPU 154, a GPU 155, a RAM 156, a ROM 157, and an external storage apparatus 158. In addition, a liquid crystal display 161 as the display unit 160, and a mouse 171 and a keyboard 172 as the input unit 170 are connected to the computer 150.

In addition, the computer 150 and the receiving unit 120 may be provided in a configuration in which the computer 150 and the receiving unit 120 are housed in a common housing. Alternatively, a part of signal processing may be performed by a computer housed in a housing and remaining signal processing may be performed by a computer provided outside of the housing. In this case, the computers provided inside and outside the housing can be collectively considered the computer according to the present embodiment. In other words, hardware constituting the computer need not be housed in a single housing. As the computer 150, an information processing apparatus provided by a cloud computing service or the like and installed at a remote location may be used.

The computer 150 corresponds to the processing unit according to the present invention. In particular, the calculating unit 151 plays a central role in realizing functions of the processing unit.

Display Unit 160

The display unit 160 is a display such as a liquid crystal display and an organic Electro Luminescence (EL) display. The display unit 160 is an apparatus which displays an image, a numerical value of a specific position, and the like based on object information and the like obtained by the computer 150. The display unit 160 may display a GUI for operating images and the apparatus. Image processing (adjustment of a brightness value and the like) may be performed by the display unit 160 or the computer 150.

Input Unit 170

As the input unit 170, an operation console which is constituted by a mouse, a keyboard, and the like and which can be operated by the user can be adopted. Alternatively, the display unit 160 may be constituted by a touch panel, in which case the display unit 160 may be used as the input unit 170. The input unit 170 accepts input of instructions, numerical values, and the like from the user and transmits the input to the computer 150.

Moreover, each component of the photoacoustic apparatus may be respectively configured as a separate apparatus or may be configured as a single integrated apparatus. Alternatively, at least a part of the components of the photoacoustic apparatus may be configured as a single integrated apparatus.

In addition, using the controlling unit 153, the computer 150 also performs drive control of the components included in the photoacoustic apparatus. Furthermore, the display unit 160 may display a GUI and the like in addition to images generated by the computer 150. The input unit 170 is configured so as to accept input of information by the user. Using the input unit 170, the user can perform operations such as starting and ending a measurement and issuing an instruction to save a created image.

Object 100

Although the object 100 does not constitute the photoacoustic apparatus, a description thereof will be given below. The photoacoustic apparatus according to the present embodiment can be used for the purposes of diagnosing a malignant tumor, a vascular disease, and the like, performing a follow-up observation of chemotherapy, and the like of a human or an animal. Therefore, as the object 100, a diagnostic subject site such as a living organism or, more specifically, breasts, respective internal organs, the vascular network, the head, the neck, the abdominal area, and the extremities including fingers and toes of a human or an animal is assumed. For example, when the measurement subject is a human body, a subject of a light absorber may be oxyhemoglobin, deoxyhemoglobin, a blood vessel containing oxyhemoglobin or deoxyhemoglobin in a large amount, or a new blood vessel formed in a vicinity of a tumor. In addition, the subject of a light absorber may be a plaque on a carotid artery wall or the like. Furthermore, pigments such as methylene blue (MB) and indocyanine green (ICG), gold particulates, or an externally introduced substance which accumulates or which is chemically modified with such pigments or gold particulates may be used as a light absorber. Moreover, a puncture needle or a light absorber added to a puncture needle may be considered an observation object. The object may be an inanimate matter such as a phantom and a product under test.

Operations of Embodiment

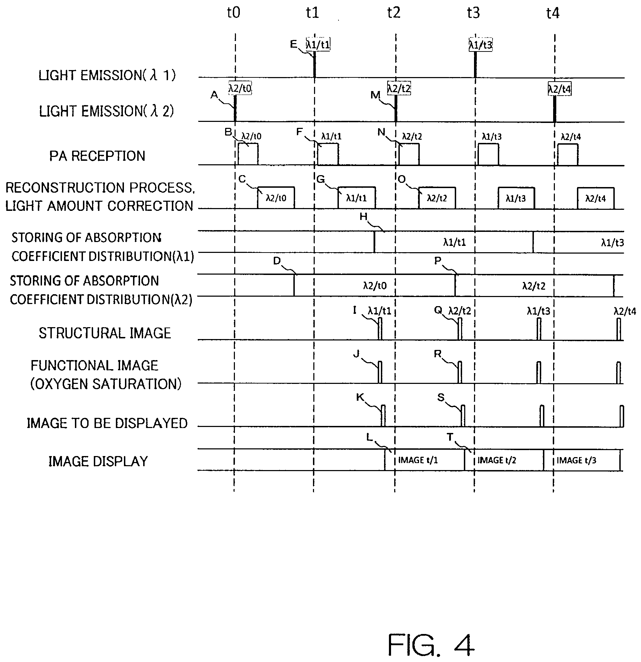

FIG. 4 is a timing chart for explaining operations in the first embodiment. In FIG. 4, a horizontal axis is a time axis. The various controls described below are basically performed by the computer 150. Notations such as "$\lambda 2/t0$" in the following description represent "emitted wavelength/light emission timing".

Timings A to D: Light Emission ($\lambda 2$), Reconstruction

The light source unit 200 emits light at the second wavelength $\lambda 2$ at a time point t0 (A). A photoacoustic wave (a second photoacoustic wave) from the object 100 is converted into a digital signal (a second signal) by the receiving unit 120 and the signal collecting unit 140 and stored in the storage unit 152 of the computer 150 (B). Subsequently, using the digital signal (the second signal) based on the photoacoustic wave stored in the storage unit 152, the computer 150 calculates an initial sound pressure distribution image (a second initial sound pressure distribution) with a reconstruction algorithm which converts the digital signal (the second signal) into three-dimensional volume data. In addition, the initial sound pressure distribution image is corrected by a light amount distribution (a second light amount distribution) of light at the second wavelength $\lambda 2$ to obtain an absorption coefficient distribution image (a second absorption coefficient distribution) (C). The absorption coefficient distribution image is stored in the storage unit 152 until acquisition of an absorption coefficient distribution image in accordance with a next light emission (at a time point t2) at the second wavelength $\lambda 2$ by the light source unit 200 (D).

Timings E to H: Light Emission ($\lambda 1$), Reconstruction

Next, the light source unit 200 emits light at the first wavelength $\lambda 1$ at a time point t1 (E). A photoacoustic wave (a first photoacoustic wave) from the object 100 is converted into a digital signal (a first signal) by the receiving unit 120 and the signal collecting unit 140 and stored in the storage unit 152 of the computer 150 (F). Subsequently, using the digital signal (the first signal) based on the photoacoustic wave stored in the storage unit 152, the computer 150 calculates an initial sound pressure distribution image (a first initial sound pressure distribution) with a reconstruction algorithm which converts the digital signal (the first signal) into three-dimensional volume data. In addition, the initial sound pressure distribution image is corrected by a light amount distribution (a first light amount distribution) of light at the first wavelength $\lambda 1$ to obtain an absorption coefficient distribution image (a first absorption coefficient distribution) (G). The absorption coefficient distribution image is stored in the storage unit 152 until acquisition of an absorption coefficient distribution image in accordance with a next light emission (at a time point t3) at the first wavelength $\lambda 1$ by the light source unit 200 (H).

Timings J to L: Generation and Display of Oxygen Saturation Distribution

Next, the computer 150 obtains an oxygen saturation distribution image which is an image (a functional image) based on functional information from the absorption coefficient distribution image (D) at the second wavelength $\lambda 2$ and the absorption coefficient distribution image (H) at the first wavelength $\lambda 1$ (J). At this point, due to a body motion of the object or a movement of the receiving unit 120, an acquisition position of a photoacoustic wave derived from the light at the first wavelength may become displaced from an acquisition position of a photoacoustic wave derived from the light at the second wavelength. In order to correct the positional displacement, the computer 150 favorably estimates a movement amount of the probe using a correlation between the obtained absorption coefficient distribution images and aligns positions of the absorption coefficient distribution image (H) and the absorption coefficient distribution image (D), and subsequently obtains an oxygen saturation distribution image with high accuracy. Alternatively, the probe 180 may be mounted with a gyro in order to measure a movement amount of the probe from the time point t0 to the time point t1.
As an alternative correcting method, the absorption coefficient distribution image (H) and the absorption coefficient distribution image (D) may be subjected to a blurring process using a smoothing (moving average) filter, a Gaussian filter, or a median filter. Performing a blurring process enables oxygen saturation which is functional information to be accurately calculated within a region of a blood vessel position even when the alignment of the positions of the absorption coefficient distribution image (H) and the absorption coefficient distribution image (D) cannot be performed in a favorable manner. Since such processing is performed, the obtained oxygen saturation distribution image (J) is often a blurred image.

Meanwhile, the computer 150 acquires a vascular image which is an image (a structural image) based on structural information by subjecting an absorption coefficient distribution image to image processing (I). An example of an image processing method involves performing binarization using a 30-percent value of a maximum absorption coefficient in the absorption coefficient distribution image as a threshold and determining a position indicating a larger absorption coefficient value than the threshold as a blood vessel.

Next, the computer 150 trims the oxygen saturation distribution image (J) with the vascular image (I) to generate a display image which presents oxygen saturation in the vascular image in a recognizable manner (K). The processing described above is completed before the next light emission (at t2) by the light source unit 200 and the obtained display image is output to the display unit 160 (L).

Timings M to P: Light Emission ($\lambda$2), Reconstruction

Next, in a similar manner to the time point t0, the light source unit 200 emits light at the second wavelength $\lambda$2 at a time point t2 (M). A photoacoustic wave from the object 100 is converted into a digital signal by the receiving unit 120 and the signal collecting unit 140 and stored in the storage unit 152 of the computer 150 (N). Subsequently, using the digital signal based on the photoacoustic wave stored in the storage unit 152, the computer 150 calculates an initial sound pressure distribution image with a reconstruction algorithm which converts the digital signal into three-dimensional volume data. In addition, the initial sound pressure distribution image is corrected by a light amount distribution of light at the second wavelength $\lambda$2 to obtain an absorption coefficient distribution image (O). The absorption coefficient distribution image is stored in the storage unit 152 until acquisition of an absorption coefficient distribution image in accordance with a next light emission (at a time point t4) by the light source unit 200 (P).

Timings Q to T: Generation and Display of Oxygen Saturation Distribution

Next, the computer 150 obtains an oxygen saturation distribution image which is a functional image from the absorption coefficient distribution image (H) at the first wavelength $\lambda$1 and the absorption coefficient distribution image (P) at the second wavelength $\lambda$2 (R). As described earlier, the obtained oxygen saturation distribution image (R) is often a blurred image. Meanwhile, the computer 150 obtains a vascular image by subjecting the absorption coefficient distribution image to image processing such as threshold processing (Q). Next, the oxygen saturation distribution image (R) is trimmed with the vascular image (Q) to generate a display image which presents oxygen saturation in the vascular image in a recognizable manner (S). The processing described above is completed before the next light emission (at t3) by the light source unit 200 and the obtained display image is output to the display unit 160 (T).

As described above, an image indicting oxygen saturation can be displayed in real time by sequentially completing a generation process of a display image derived from a given pair of light irradiations before a next light irradiation by the light source unit 200 and outputting the obtained display image to the display unit 160. In this case, using the absorption coefficient distribution image (H) at the first wavelength $\lambda$1 for both the generation of the display image (L) and the generation of the display image (T), a frame of a display image can be generated for each light emission without having to wait for light emission at the two wavelengths to be completed.

With the object information acquiring method in accordance with the timing chart described above, a plurality of light emissions are performed at two wavelengths (the first and second wavelengths). In the plurality of light emissions, either one of the wavelengths may be irradiated first. For example, a light emission at t0 may be referred to as a first irradiation, a light emission at t1 may be referred to as a second irradiation, a light emission at t2 may be referred to as a third irradiation, and so forth, or a light emission at t1 may be referred to as a first irradiation, a light emission at t2 may be referred to as a second irradiation, a light emission at t3 may be referred to as a third irradiation, and so forth.

In addition, a signal derived from a photoacoustic wave excited by the first irradiation can be referred to as a first signal, a signal derived from a photoacoustic wave excited by the second irradiation can be referred to as a second signal, a signal derived from a photoacoustic wave excited by the third irradiation can be referred to as a third signal, and so forth.

Furthermore, an absorption coefficient distribution based on the first signal can be referred to as a first absorption coefficient distribution, an absorption coefficient distribution based on the second signal can be referred to as a second absorption coefficient distribution, an absorption coefficient distribution based on the third signal can be referred to as a third absorption coefficient distribution, and so forth.

In addition, structural information extracted from an absorption coefficient distribution based on the first signal can be referred to as first structural information, structural information extracted from an absorption coefficient distribution based on the second signal can be referred to as second structural information, structural information extracted from an absorption coefficient distribution based on the third signal can be referred to as third structural information, and so forth.

Furthermore, functional information generated from an absorption coefficient distribution based on the first signal and an absorption coefficient distribution based on the second signal can be referred to as first functional information, functional information generated from an absorption coefficient distribution based on the second signal and an absorption coefficient distribution based on the third signal can be referred to as second functional information, and so forth.

In addition, an image obtained by trimming the first functional information based on at least any of the first to third structural information (favorably, the first or second structural information) can be referred to as a first display image, an image obtained by trimming the second functional information based on at least any of the first to third structural information (favorably, the second or third structural information) can be referred to as a second display image, and so on.

With the object information acquiring method in accordance with the timing chart described above, the number of times a display image is generated increases as compared to a conventional method in which a display image is obtained after light emission at a plurality of wavelengths is finished. Therefore, the number of times a display image is updated relative to the number of light emissions increases as compared to conventional art. As a result, time trackability with respect to probe movement is improved.

For example, when a "first number of light emissions" refers to the number of light emissions at the first wavelength related to the first absorption coefficient distribution used in order to generate an oxygen saturation distribution and a "second number of light emissions" refers to the number of light emissions at the second wavelength related to the second absorption coefficient distribution used in order to generate the oxygen saturation distribution, then, with conventional methods, "number of generations of display image = first number of light emissions = second number of light emissions".

On the other hand, in the method in accordance with the timing chart described above, since an absorption coefficient distribution used to generate a display image in a given frame is also used to generate a display image in a subsequent frame, "number of generations of display image = first number of light emissions + second number of light emissions". Sequentially displaying this display image realizes smooth real-time display which only provides a small sense of incongruity.

Moreover, in the case of the timing chart described above, the display image is sequentially updated each time light is emitted at the first or second wavelength. However, even when a display image is not generated each time light is emitted, the effects of the present invention may be produced as long as the number of times a display image is generated exceeds that of conventional art. This relationship can be expressed as a case where the number of times a display image is generated satisfies expressions (1) to (3) below.

```
First number of light emissions < number of generations ...

(1)

 Second number of light emissions < number of generations

 ... (2)

 Number of generations ≤ first number of light emissions +

 second number of light emissions ... (3)
```

Flow Chart

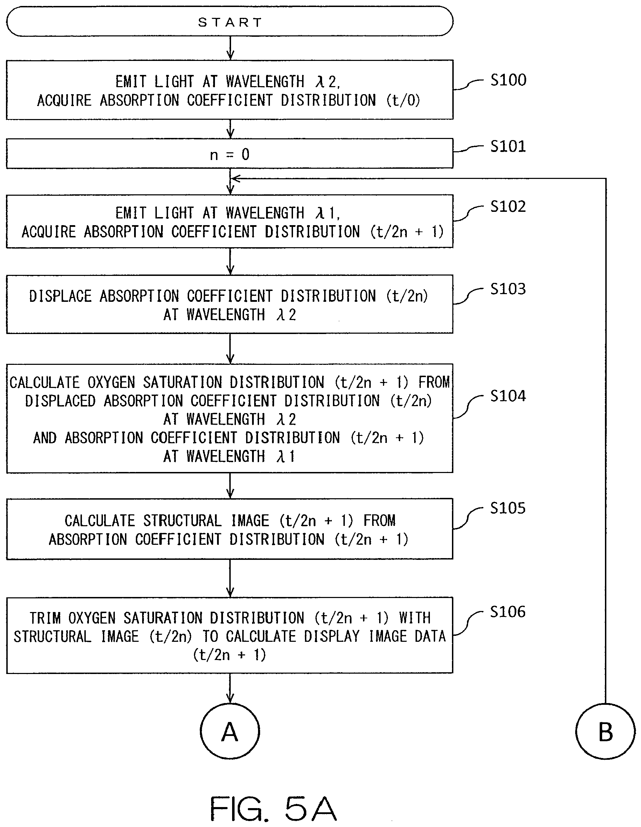

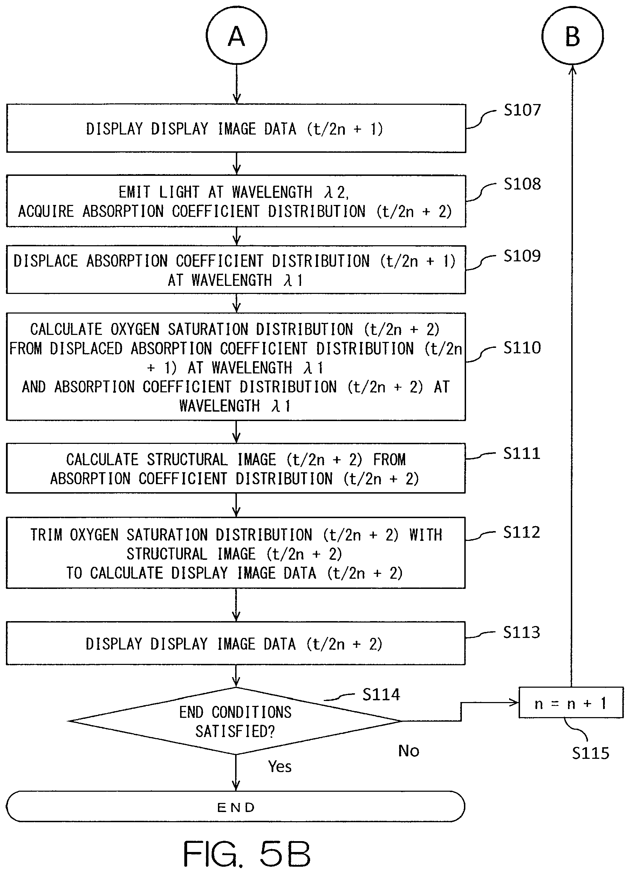

FIGS. 5A and 5B are flow charts for explaining operations in the first embodiment of the present invention. Although some portions overlap with the description given with reference to the timing chart shown in FIG. 4, operations performed by the computer 150 will now be described with reference to FIGS. 5A and 5B.

In step S100, the light source unit 200 emits light at a wavelength λ2 and acquires an absorption coefficient distribution image at the wavelength λ2 (FIG. 4, A to D).

In step S101, the computer 150 assigns "0" to a variable "n".

In step S102, the light source unit 200 emits light at a wavelength λ1 and acquires an absorption coefficient distribution image at the wavelength $\lambda 1$ (FIG. 4, E to H).

In step S103, the computer 150 displaces the absorption coefficient distribution image at the wavelength $\lambda 2$.

In step S104, an oxygen saturation distribution image is calculated from the displaced absorption coefficient distribution image at the wavelength $\lambda 2$ and the absorption coefficient distribution image at the wavelength $\lambda 1$ obtained in step S102 (FIG. 4, J). Moreover, a method of correcting a displacement in light irradiation positions between light emission timings is arbitrary. For example, an image generation position may be aligned with an irradiation position of the wavelength $\lambda 2$ or with an intermediate position between the respective irradiation positions of the wavelengths $\lambda 1$ and $\lambda 2$. Regardless of what method is used, points indicating a same position inside the object in two images need only be associated with each other.

In step S105, the computer 150 calculates a vascular image (a structural image) from an absorption coefficient distribution image (FIG. 4, I).

In step S106, the computer 150 calculates display image data by trimming an oxygen saturation distribution with the vascular image (the structural image) (FIG. 4, K).

In step S107, the display image data is displayed (FIG. 4, L).

According to the processing described above, image processing for one unit of real-time image display is completed. A display unit is defined by light emission intervals and, in the present embodiment, one light emission corresponds to one unit regardless of the wavelength.

In step S108, the light source unit 200 emits light at the wavelength $\lambda 2$ and acquires an absorption coefficient distribution image at the wavelength $\lambda 2$ (FIG. 4, M to P).

In step S109, the computer 150 displaces the absorption coefficient distribution image at the wavelength $\lambda 1$.

In step S110, the computer 150 calculates an oxygen saturation distribution image from the displaced absorption coefficient distribution image at the wavelength $\lambda 1$ and the absorption coefficient distribution image at the wavelength $\lambda 2$ obtained in step S108 (FIG. 4, R).

In step S111, the computer 150 calculates a vascular image (a structural image) from an absorption coefficient distribution image (FIG. 4, Q).

In step S112, the computer 150 calculates display image data by trimming an oxygen saturation distribution with the vascular image (the structural image) (FIG. 4, S).

In step S113, the display image data is displayed (FIG. 4, T).

According to the processing described above, image processing for a next unit of real-time image display is completed.

In step S114, the computer 150 determines whether or not end conditions are satisfied. Conceivable examples of the end conditions include detection of a stop instruction from the user using the input unit, detection by a touch sensor or the like of a hand of the user disengaging from the probe 180, and a lapse of a prescribed amount of time from the start of measurement. When the end conditions are satisfied, photoacoustic measurement and real-time display end.

On the other hand, when the end conditions are not satisfied, the computer 150 advances to step S115. The computer 150 increments n and returns to step S102 to repeat the processes described above.

As described above, operations of the timing chart described with reference to FIG. 4 are executed by updating and displaying display image data so as to coincide with light emissions by the light source unit 200 in accordance with the present flow chart.

With a display method using the photoacoustic apparatus according to the present embodiment, when acquiring oxygen saturation using light of a plurality of wavelengths that differ from each other, a display image can be formed by accurately obtaining a structural image (a vascular image) and a functional image within irradiation intervals of a light pulse and the display image can be displayed in real time.

First Modification

In the processes of steps S105 and S111 in the flow chart shown in FIG. 5, vascular images (structural information) are obtained by providing a threshold and performing a binarization process on absorption coefficient distribution images respectively corresponding to the first and second wavelengths. In the present modification, a method of correcting an error in a blood vessel width to obtain a preferable vascular image (structural information) will be described.

Errors in Vascular Image

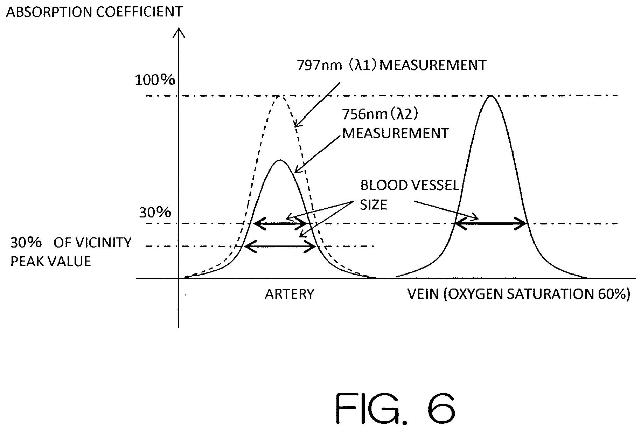

As a premise for viewing FIG. 6, it is assumed that an artery and a vein run inside an object that is a subject of photoacoustic measurement and that both blood vessels have similar widths. FIG. 6 respectively shows, with respect to the object described above, a dashed line depicting an absorption coefficient distribution generated from an acoustic wave derived from the first wavelength $\lambda 1$ (797 nm) and a solid line depicting an absorption coefficient distribution derived from the second wavelength $\lambda 2$ (756 nm). A left side of the drawing corresponds to an artery with relatively high oxygen saturation and a right side of the drawing corresponds to a vein with relatively low oxygen saturation (for example, around 60%). Moreover, a graph of the first wavelength and a graph of the second wavelength overlap with each other on the right side of the drawing which corresponds to the vein.

A phenomenon is known in which a blood vessel width of an artery in an extracted vascular image changes according to a wavelength of light due to a difference in absorption coefficients between the two types of hemoglobin.

As described earlier, at the first wavelength $\lambda 1$, absorption coefficients of oxyhemoglobin and deoxyhemoglobin are substantially equal to each other. Therefore, as shown in FIG. 6, in an absorption coefficient distribution image derived from the first wavelength, a blood vessel with a same size is extracted in a same size regardless of whether the blood vessel is an artery or a vein. Accordingly, when extracting a blood vessel from an absorption coefficient distribution image corresponding to the first wavelength, regardless of whether the blood vessel is an artery or a vein, a vascular image (a structural image) can be preferably acquired by threshold processing (for example, a binarization process using a 30-percent value of a maximum absorption coefficient as a threshold).

On the other hand, since the absorption coefficient of deoxyhemoglobin peaks at the second wavelength $\lambda 2$, there is a large difference between the absorption coefficients of oxyhemoglobin and deoxyhemoglobin. Therefore, in an absorption coefficient distribution image derived from the second wavelength, even when blood vessel widths are the same, a vein is extracted in a larger size while an artery is extracted in a smaller size.

Correcting Method

In real-time display which is moving image display, there is a concept that a blood vessel width need not be corrected because a sense of interference imparted to the user by an error in the blood vessel width is small. However, the following method is effective when performing a correction in order to display an image with higher accuracy. First, a peak value of an absorption coefficient is acquired in a vicinity of a region of interest in an absorption coefficient distribution image. The vicinity may be determined based on the number of picture elements such as pixels and voxels or determined based on distance. Subsequently, a peak value (a vicinity peak value) in a vicinity of a region of interest is acquired, a 30-percent value of the vicinity peak value is set as a second threshold, and a portion having a higher absorption coefficient value than the second threshold is extracted as a blood vessel. Generally, since a vicinity peak value is smaller than a peak value of an entire image, the number of pixels extracted as a blood vessel increases and an artery with a large blood vessel width is extracted.

Second Modification

In addition, the structural image (the vascular image) obtained at the wavelength $\lambda 1$ may be corrected and used as the structural image (the vascular image) at the wavelength $\lambda 2$. When performing correction, a calculation based on a movement of a probe or a body motion is performed. However, when an irregular motion such as a body motion occurs, the structural image at the wavelength $\lambda 1$ must be subjected to complex processing (for example, processing involving dividing an image and obtaining and correcting a motion in each divided region) which calls for an enormous amount of calculations. In addition, when there is an error in motion estimation, an unnatural moving image is produced. Therefore, for real-time display which requires a display image to be created in a limited period of time, a method of obtaining a structural image (a vascular image) from an absorption coefficient distribution image obtained upon each irradiation of a light pulse with a different wavelength by the light source unit 200 is preferable since the method enables natural display to be performed with a simple configuration.

Second Embodiment

When obtaining a structural image (a vascular image) by threshold processing with respect to an absorption coefficient distribution image at a wavelength with a large difference in absorption coefficients between the two types of hemoglobin, as shown in FIG. 6, there is a possibility that a difference may arise among blood vessel widths extracted in accordance with oxygen saturation. In a second embodiment, a method of obtaining a structural image (a vascular image) with high accuracy even from such an absorption coefficient distribution image will be described. It should be noted that descriptions of same components and same processing as the first embodiment will be omitted.

In step S105 shown in FIG. 5, the wavelength $\lambda 1$ (797 nm) used to calculate a vascular image (a structural image) from an absorption coefficient distribution image is a wavelength at which absorption coefficients of the two type of hemoglobin are substantially equal to each other. Therefore, by determining a threshold based on a peak value in the absorption coefficient distribution image and performing a binarization process, structural information (a blood vessel width) can be preferably determined.

On the other hand, at the second wavelength $\lambda 2$ (756 nm) used to extract a blood vessel in step S111, absorption coefficients differ between the two types of hemoglobin. Therefore, acquiring structural information (a blood vessel width) using the same method as S105 results in extracting a different blood vessel width even when an artery and a vein actually have a same blood vessel width. In order to address this issue, in the first modification of the first embodiment, structural information (a blood vessel width) is acquired in the process of step S111 by determining a threshold (the second threshold) based on a value of a peak in a vicinity of a region of interest and performing a binarization process.

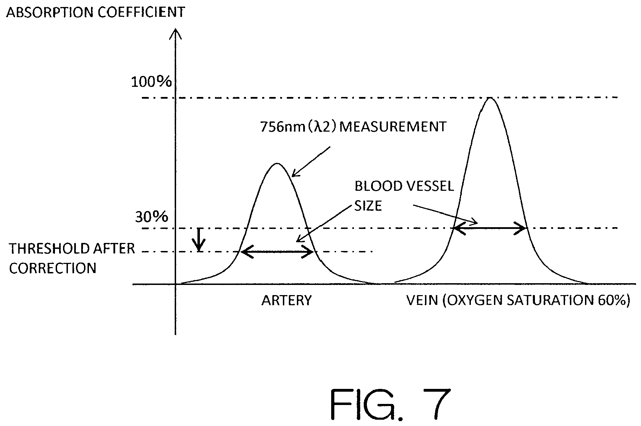

In the second embodiment, in order to calculate structural information (a blood vessel width) with high accuracy, a threshold to be used in step S111 is corrected using, additionally, an oxygen saturation distribution image obtained in step S110 which is a functional image. Specifically, first, the computer 150 extracts a region with high oxygen saturation such as an artery from the oxygen saturation distribution image obtained in step S110. Next, the extracted region is corrected so as to lower the threshold. On the other hand, the threshold is not corrected with respect to a region with low oxygen saturation such as a vein. This situation is shown in FIG. 7. FIG. 7 shows that, in an artery image at the second wavelength, the threshold has been reduced from 30% and is now around 20%. Using a threshold (a third threshold) corrected in this manner enables a correct structural image (a vascular image) to be acquired regardless of the wavelength.

Methods of correcting a threshold include, for instance, a method of obtaining a new threshold by multiplying an original threshold by a constant proportional to oxygen saturation and a method of storing correction values corresponding to oxygen saturation in a table and obtaining a new threshold by multiplying an original threshold by a correction value referred to based on oxygen saturation. Alternatively, a threshold (for example, what percentage of a peak is to be adopted as a threshold) itself corresponding to oxygen saturation may be directly stored in a table and a threshold may be determined by referring to the threshold based on oxygen saturation. Such methods enable an appropriate threshold to be acquired in a simple manner based on an oxygen saturation distribution image.

In addition, in the first embodiment, a light source with a wavelength at which a difference between the absorption coefficients of the two types of hemoglobin is small has been used as the wavelength $\lambda 1$. However, when performing the threshold correction described above, a wavelength with a small difference in absorption coefficients need not necessarily be used as the wavelength $\lambda 1$.

According to the second embodiment, a display image can be formed by accurately obtaining a structural image and a functional image within irradiation intervals of light pulses of the light source unit 200 with different wavelengths and the display image can be displayed in real time.

Third Embodiment

Next, a third embodiment will be described. In the first embodiment, a display image is updated for each irradiation of a light pulse with a different wavelength. This mode will be referred to as a "real-time mode" for the sake of convenience. A photoacoustic apparatus according to the third embodiment includes a "fidelity mode" in which display is performed with higher fidelity when display in the "real-time mode" is not favorable, and enables switching between the respective modes. It should be noted that descriptions of same components and same processing as the respective embodiments described above will be omitted.

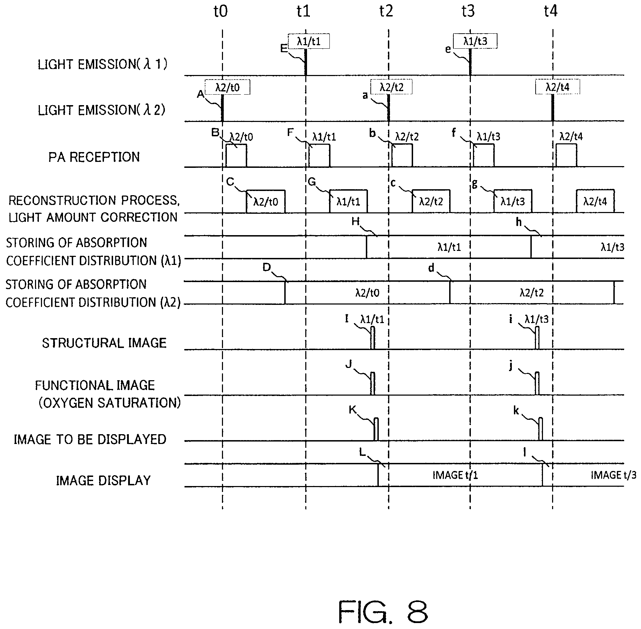

FIG. 8 is a timing chart of a mode (the "fidelity mode") which enables a structural image to be displayed with high accuracy according to the third embodiment of the present invention. In FIG. 8, a horizontal axis is a time axis. Controls are performed by the computer 150.

In a similar manner to the first embodiment, first, an absorption coefficient distribution image is acquired based on light at the second wavelength $\lambda 2$ which is emitted by the light source unit 200 at a time point t0 (A to C). The absorption coefficient distribution image is stored in the storage unit 152 until acquisition of an absorption coefficient distribution image in accordance with a next light emission (at a time point t2) at the second wavelength $\lambda 2$ by the light source unit 200 (D). Next, an absorption coefficient distribution image is acquired based on light at the first wavelength $\lambda 1$ which is emitted by the light source unit 200 at a time point t1 (E to G). The absorption coefficient distribution image is stored in the storage unit 152 until acquisition of an absorption coefficient distribution image in accordance with a next light emission (at a time point t3) by the light source unit 200 (H).

Subsequently, in a similar manner to the first embodiment, an oxygen saturation distribution image which is a functional image is obtained from the absorption coefficient distribution image (D) at the second wavelength $\lambda 2$ and the absorption coefficient distribution image (H) at the first wavelength $\lambda 1$ (J). On the other hand, a structural image (a vascular image) is acquired by image processing (for example, a binarization process using a 30%-value of a maximum absorption coefficient as a threshold) on an absorption coefficient distribution image (I). Next, the oxygen saturation distribution image (J) is trimmed with the vascular image (I) to calculate a display image which presents oxygen saturation in the vascular image in a recognizable manner (K). In addition, the obtained display image is output to the display unit 160 (L).

Next, an absorption coefficient distribution image is acquired based on light at the second wavelength $\lambda 2$ which is emitted by the light source unit 200 at a time point t2 (a to c). The absorption coefficient distribution image is stored in the storage unit 152 until acquisition of an absorption coefficient distribution image in accordance with a next light emission (at a time point t4) at the second wavelength λ2 by the light source unit 200 (d). Next, an absorption coefficient distribution image is acquired based on light at the first wavelength λ1 which is emitted by the light source unit 200 at a time point t3 (e to g). The absorption coefficient distribution image is stored in the storage unit 152 until acquisition of an absorption coefficient distribution image in accordance with a next light emission (at a time point t5) by the light source unit 200 (h).

More specifically, in the present embodiment, generation of a display image corresponding to (S) in FIG. 4 is not performed. In other words, threshold processing and trimming using an absorption coefficient distribution image corresponding to the second wavelength λ2 are not performed.

Subsequently, the computer 150 obtains an oxygen saturation distribution image which is a functional image from the absorption coefficient distribution image (d) at the second wavelength λ2 and the absorption coefficient distribution image (h) at the first wavelength λ1 (j). Meanwhile, the computer 150 obtains a structural image (a vascular image) by subjecting the absorption coefficient distribution image to threshold processing (for example, trimming using a 30%-value of a maximum absorption coefficient as a threshold) (i). Next, the oxygen saturation distribution image (j) is trimmed with the vascular image (i) to generate a display image which presents oxygen saturation in the vascular image in a recognizable manner (k). In addition, the obtained display image is output to the display unit 160 (1).

By sequentially performing the processing described above for each pair of light irradiations at the wavelengths λ2 and λ1 by the light source unit 200 and outputting an obtained display image to the display unit 160, the display image can be displayed in real time. In this case, the display image is updated by the display unit 160 for each pair of light irradiations at the wavelengths λ2 and λ1.

As described above, in the "fidelity mode", since only trimming at the first wavelength with no difference in absorption coefficients between the two types of hemoglobin is performed, the accuracy of a structural image is improved. Therefore, a variation in blood vessel width in a display image is eliminated. On the other hand, an update frequency of a display image by the display unit 160 in the "fidelity mode" decreases to 1/2 of that in the "real-time mode" and produces a display with a low refresh frequency. As a result, display following a motion of the probe 180 imparts a sense of interference such as delay and jumpiness.

The photoacoustic apparatus according to the present embodiment has two modes, namely, the "real-time mode" which provides high trackability to probe movement but only offers structural images with low accuracy and the "fidelity mode" which offers structural images with high accuracy but only provides low trackability. There are various conceivable methods of specifying a mode or switching between modes in such a photoacoustic apparatus. For example, the user may specify a mode via the input unit 170 in accordance with the user's preference or a state of the object. Alternatively, the computer 150 may automatically switch between modes in accordance with a motion of the probe 180. Specifically, the photoacoustic apparatus may be controlled so as to automatically switch to the "fidelity mode" when a speed of the probe 180 is low (or when the probe 180 stops) and to the "real-time mode" when the speed of the probe 180 is high. Methods of detecting speed include a method involving mounting the probe 180 with a gyro and a method involving obtaining speed by calculating an amount of movement using a correlation between light emission intervals of the light source unit 200 and absorption coefficient distribution images. In addition, particularly when modes are switched automatically, a current mode is favorably displayed using characters, an icon, or the like on the display unit 160 to inform the user.

According to the third embodiment of the present invention, two modes can be used while switching between the modes either in accordance with the user's intention or automatically. As a result, a structural image is displayed with higher accuracy when a motion of the probe 180 is slow, and display which tracks motion with a high refresh frequency can be performed when the motion of the probe 180 is fast.

Other Embodiments

Modification Related to Probe and Light Source Unit

The present invention can be realized even with a configuration in which the light source unit 200 is provided inside the probe 180. The probe 180 according to the present modification internally includes the light source unit 200 made up of a light source constituted by a laser diode (LD) and a light-emitting diode (LED) and a driver circuit. In order to measure oxygen saturation, the light source unit 200 is favorably configured by combining a plurality of LDs and LEDs with different emission wavelengths. The optical system 112 according to the present modification is an optical system such as an optical fiber, a lens, or a mirror optical system which guides light pulses from the LD and the LED to the light irradiating unit 113 inside the probe 180. Alternatively, a structure may be adopted in which the object 100 is directly irradiated with light pulses from the LD and the LED without involving the optical members.

In the case of a configuration which integrates the light source unit 200 with the probe 180 as in the present modification, it is difficult to reduce light emission intervals by the light source due to the problem of heat generation. However, according to the present invention, since the light emission frequency of a light source and a refresh frequency of a display image can be set the same, preferable real-time display can be achieved.

Modification Related to Irradiation Timing

A mode in which light at two wavelengths are alternately emitted has been described in the respective embodiments presented above. However, the present invention can be applied to light emission systems other than alternate light emission. For example, a light emission system is conceivable in which, after repeating light emission at the first wavelength λ1 twice, light emission at the second wavelength λ2 is repeated twice. In this case, the computer 150 first generates an absorption coefficient distribution image based on a photoacoustic wave obtained at a given light emission timing (a timing 1) and obtains structural information by threshold processing. Subsequently, an absorption coefficient distribution image derived from light emission at the timing 1, and a timing (a timing 2) near the timing 1 at which light at a different wavelength had been emitted, are detected. Next, a functional image (an oxygen saturation distribution image) is obtained using the absorption coefficient distribution image derived from light emission at the timing 1 and an absorption coefficient distribution image derived from light emission at the timing 2. Subsequently, a display image is generated using the structural information obtained earlier.

According to the present modification, preferable real-time display can be realized even in cases other than alternate irradiations of two wavelengths.

Modification Related to Number of Wavelengths

Although two wavelengths have been used in the respective embodiments described above, the present invention can also be applied to cases where light of at least three wavelengths are sequentially emitted. In this case, shape information may be obtained from an absorption coefficient distribution image in accordance with light emission at each time point regardless of wavelength, and a functional image may be obtained using the absorption coefficient distribution image in accordance with light emission at each time point and an absorption coefficient distribution image in accordance with light emission at a different wavelength at a timing near the time point. When necessary, a functional image may be obtained using an absorption coefficient distribution image in accordance with light emission at each time point regardless of wavelength and absorption coefficient distribution images in accordance with light emissions at a plurality of different wavelengths at timings near the time point.

Modification Related to Display Image

In the respective embodiments presented above, a method in which, after extracting a position of a blood vessel inside an object, an oxygen saturation distribution is trimmed based on the position of the blood vessel has been described as a method of processing functional information based on structural information to generate a display image. However, methods of image processing are not limited thereto. For example, an image based on structural information and an image based on functional information may be displayed superimposed on one another. In addition, when displaying an image based on structural information and an image based on functional information superimposed on one another, the images may be respectively displayed in different hues in order to distinguish the images from each other and to improve visibility.

Furthermore, the present invention can also be realized by executing the processing described below. Specifically, the present invention can also be realized by supplying software (a program) that realizes functions of the embodiments described above to a system or an apparatus via a network or various storage media and having a computer (or a CPU, an MPU, or the like) in the system or the apparatus read and execute the program.

Other Embodiments

Embodiment(s) of the present invention can also be realized by a computer of a system or apparatus that reads out and executes computer executable instructions (e.g., one or more programs) recorded on a storage medium (which may also be referred to more fully as a 'non-transitory computer-readable storage medium') to perform the functions of one or more of the above-described embodiment(s) and/or that includes one or more circuits (e.g., application specific integrated circuit (ASIC)) for performing the functions of one or more of the above-described embodiment(s), and by a method performed by the computer of the system or apparatus by, for example, reading out and executing the computer executable instructions from the storage medium to perform the functions of one or more of the above-described embodiment(s) and/or controlling the one or more circuits to perform the functions of one or more of the above-described embodiment(s). The computer may comprise one or more processors (e.g., central processing unit (CPU), micro processing unit (MPU)) and may include a network of separate computers or separate processors to read out and execute the computer executable instructions. The computer executable instructions may be provided to the computer, for example, from a network or the storage medium. The storage medium may include, for example, one or more of a hard disk, a random-access memory (RAM), a read only memory (ROM), a storage of distributed computing systems, an optical disk (such as a compact disc (CD), digital versatile disc (DVD), or Blu-ray Disc (BD)™), a flash memory device, a memory card, and the like.

While the present invention has been described with reference to exemplary embodiments, it is to be understood that the invention is not limited to the disclosed exemplary embodiments. The scope of the following claims is to be accorded the broadest interpretation so as to encompass all such modifications and equivalent structures and functions.

**Claims**

1. An object information acquiring apparatus, comprising:

a light irradiating unit configured to irradiate an object with light at a first wavelength and with light at a second wavelength which differs from the first wavelength;
a receiving unit configured to receive an acoustic wave propagated from the object and to output a signal;
a processing unit configured to generate, based on the acoustic wave, structural information and functional information on the object; and
a display controlling unit configured to cause a display image based on the structural information and the functional information to be displayed on a display unit, wherein
the light irradiating unit is configured to:

perform a first irradiation in which the object is irradiated with the light at a first wavelength,
perform a second irradiation in which the object is irradiated with the light at a second wavelength after the first irradiation, and
perform a third irradiation in which the object is irradiated with the light at a first wavelength after the second irradiation,

the receiving unit is configured to receive the acoustic wave derived from each of the first to third irradiations and output first to third signals,
the processing unit is configured to:

generate at least any of first to third structural information respectively based on the first to third signals;
generate first functional information based on the first and second signals and generates second functional information based on the second and third signals; and
generate first and second display images respectively based on the first and second functional information, at least any of the first to third structural information being used to generate the first and second display images, and

the display controlling unit is configured to cause the first and second display images to be sequentially displayed on the display unit.

2. The object information acquiring apparatus according to claim 1, wherein
the processing unit is configured to:

generate at least the second and third structural information among the first to third structural information; and
generate the first display image based on the first functional information and the second structural information and generate the second display image based on the second functional information and the third structural information.

3. The object information acquiring apparatus according to claim 1 or 2, wherein
the functional information indicates an oxygen saturation distribution of the object and the structural information indicates a structure of a blood vessel in the object, and
the processing unit is configured to generate the display image by trimming the oxygen saturation distribution using the structure of the blood vessel.

4. The object information acquiring apparatus according to claim 3, wherein
one of the first and second wavelengths is a wavelength at which absorption coefficients of oxyhemoglobin and deoxyhemoglobin are substantially equal to each other and the other of the first and second wavelengths is a wavelength at which the absorption coefficients of oxyhemoglobin and deoxyhemoglobin differ from each other, and the processing unit is configured to:

generate a first absorption coefficient distribution based on the first signal, generate a second absorption coefficient distribution based on the second signal, generate the oxygen saturation distribution using the first and second absorption coefficient distributions; and
generate the structural information by threshold processing performed on at least any of the first and second absorption coefficient distributions.

5. The object information acquiring apparatus according to claim 4, wherein
when generating the structural information using an absorption coefficient distribution derived from a wavelength at which the absorption coefficients of oxyhemoglobin and deoxyhemoglobin differ from each other among the first and second wavelengths, the processing unit is configured to perform correction so that a width of a blood vessel, which is an artery, increases.

6. The object information acquiring apparatus according to claim 1 or 2, wherein
the display controlling unit is configured to cause an image based on the structural information and an image based on the functional information to be displayed superimposed on each other on the display unit.

7. The object information acquiring apparatus according to claim 6, wherein
the display controlling unit is configured to cause an image based on the structural information and an image based on the functional information to be displayed in different hues on the display unit.

8. The object information acquiring apparatus according to any one of claims 1 to 7, wherein
positions at which the receiving unit receives the acoustic waves respectively derived from the first to third irradiations differ from each other.

9. The object information acquiring apparatus according to claim 8, wherein
the receiving unit is arranged inside a handheld type probe.

10. The object information acquiring apparatus according to claim 8, further comprising
a scanning unit configured to move the receiving unit.

11. The object information acquiring apparatus according to any one of claims 8 to 10, wherein
the processing unit is configured to generate the first and second functional information using positions at which the receiving unit has received acoustic waves respectively derived from the first to third irradiations.

12. An object information acquiring apparatus, comprising:

a light irradiating unit configured to irradiate an object with light at a first wavelength and with light at a second wavelength, which differs from the first wavelength;
a receiving unit configured to receive an acoustic wave propagated from the object and outputs a signal;
a processing unit configured to perform reconstruction based on the acoustic wave; and
a display controlling unit configured to cause a display image based on image data generated by the reconstruction to be displayed on a display unit, wherein
the processing unit is configured to:

generate an oxygen saturation distribution from a first absorption coefficient distribution reconstructed from an acoustic wave derived from the light at a first wavelength and a second absorption coefficient distribution reconstructed from an acoustic wave derived from the light at a second wavelength; and
generate the oxygen saturation distribution for the number of generations that satisfying expressions (1) to (3) given below when (A) the number of light emissions at the first wavelength related to the first absorption coefficient distribution used in order to generate the oxygen saturation distribution is defined as a first number of light emissions and (B) the number of light emissions at the second wavelength related to the second absorption coefficient distribution used in order to generate the oxygen saturation distribution is defined as a second number of light emissions, and

the display controlling unit is configured to cause an image based on the oxygen saturation distribution to be displayed as the display image on the display unit.

$$\text{First number} < \text{number of generations} \dots (1)$$

$$\text{Second number} < \text{number of generations} \dots (2)$$

$$\text{Number of generations} \leq \text{first number} + \text{second number} \dots (3).$$

13. The object information acquiring apparatus according to claim 12, wherein
the processing unit is configured to generate the oxygen saturation distribution for each light emission at the first wavelength and for each light emission at the second wavelength, and
the display controlling unit is configured to sequentially display an image based on the oxygen saturation distribution on the display unit each time the oxygen saturation distribution is generated.

14. The object information acquiring apparatus according to claim 12 or 13, wherein
the processing unit is configured to extract a blood vessel from the oxygen saturation distribution using the first absorption coefficient distribution used in order to generate the oxygen saturation distribution or the second absorption coefficient distribution used in order to generate the oxygen saturation distribution, and
the display controlling unit is configured to display the oxygen saturation distribution, from which the blood vessel has been extracted, on the display unit as the display image.

15. A display method, comprising:

acquiring a first signal derived from an acoustic wave generated from an object due to a first irradiation at a first wavelength;
acquiring a second signal derived from an acoustic wave generated from the object due to a second irradiation at a second wavelength, which differs from the first wavelength;
acquiring a third signal derived from an acoustic wave generated from the object due to a third irradiation at the first wavelength;
generating first functional information based on the first signal and the second signal;
generating second functional information based on the second signal and the third signal; and
sequentially displaying a first display image based on the first functional information and a second display image based on the second functional information.

16. The display method according to claim 15, further comprising generating at least second and third structural information from among first to third structural information respectively based on the first to third signals, and
the second structural information is used to generate the first display image and the third structural information is used to generate the second display image.

17. The display method according to claim 16, wherein
the first functional information and the second functional information indicate an oxygen saturation distribution of the object and the first structural information to the third structural information indicate a structure of a blood vessel in the object, and
the first display image and the second display image are generated by trimming the oxygen saturation distribution using the structure of the blood vessel.

18. The display method according to claim 17, wherein one of the first and second wavelengths is a wavelength at which absorption coefficients of oxyhemoglobin and deoxyhemoglobin are substantially equal to each other and the other of the first and second wavelengths is a wavelength at which the absorption coefficients of oxyhemoglobin and deoxyhemoglobin differ from each other,
the display method further comprising generating a first absorption coefficient distribution based on the first signal, generating a second absorption coefficient distribution based on the second signal, and generating the oxygen saturation distribution using the first and second absorption coefficient distributions, and
in the generating of the structural information, the structural information is generated by threshold processing performed on at least any of the first and second absorption coefficient distributions.

19. The display method according to claim 18, wherein
in the generating of the structural information, when generating the structural information using an absorption coefficient distribution derived from a wavelength at which the absorption coefficients of oxyhemoglobin and deoxyhemoglobin differ from each other among the first and second wavelengths, correction is performed so that a width of a blood vessel, which is an artery, increases.

20. The display method according to claim 16, wherein
in the sequentially displaying of the display images, an image based on at least any of the first structural information to the third structural information and an image based on at least any of the first functional information and the second functional information are displayed superimposed on each other.

21. The display method according to claim 20, wherein
in the sequentially displaying of the display images, an image based on at least any of the first structural information to the third structural information and an image based on at least any of the first functional information and the second functional information are displayed in different hues.

FIG. 1

FIG. 2

150

154

CPU

155

GPU

156

RAM

157

ROM

158

EXTERNAL STORAGE
APPARATUS

161

LIQUID CRYSTAL
DISPLAY

171

MOUSE

172

KEYBOARD

# FIG. 3

FIG. 4

```
                    ┌─────────────────────────────────┐
                    │             START               │
                    └─────────────────────────────────┘
                                    │
                                    ▼
    ┌─────────────────────────────────────────────────┐
    │             EMIT LIGHT AT WAVELENGTH λ2,         │──── S100
    │    ACQUIRE ABSORPTION COEFFICIENT DISTRIBUTION (t/0) │
    └─────────────────────────────────────────────────┘
                                    │
                                    ▼
    ┌─────────────────────────────────────────────────┐
    │                    n = 0                         │──── S101
    └─────────────────────────────────────────────────┘
                                    │
                                    ▼
    ┌─────────────────────────────────────────────────┐
    │             EMIT LIGHT AT WAVELENGTH λ1,         │──── S102
    │  ACQUIRE ABSORPTION COEFFICIENT DISTRIBUTION (t/2n + 1) │
    └─────────────────────────────────────────────────┘
                                    │
                                    ▼
    ┌─────────────────────────────────────────────────┐
    │   DISPLACE ABSORPTION COEFFICIENT DISTRIBUTION (t/2n) │──── S103
    │              AT WAVELENGTH λ2                    │
    └─────────────────────────────────────────────────┘
                                    │
                                    ▼
    ┌─────────────────────────────────────────────────┐
    │ CALCULATE OXYGEN SATURATION DISTRIBUTION (t/2n + 1) FROM │
    │   DISPLACED ABSORPTION COEFFICIENT DISTRIBUTION (t/2n) │──── S104
    │              AT WAVELENGTH λ2                    │
    │   AND ABSORPTION COEFFICIENT DISTRIBUTION (t/2n + 1) │
    │              AT WAVELENGTH λ1                    │
    └─────────────────────────────────────────────────┘
                                    │
                                    ▼
    ┌─────────────────────────────────────────────────┐
    │      CALCULATE STRUCTURAL IMAGE (t/2n + 1) FROM  │──── S105
    │    ABSORPTION COEFFICIENT DISTRIBUTION (t/2n + 1) │
    └─────────────────────────────────────────────────┘
                                    │
                                    ▼
    ┌─────────────────────────────────────────────────┐
    │  TRIM OXYGEN SATURATION DISTRIBUTION (t/2n + 1) WITH │──── S106
    │ STRUCTURAL IMAGE (t/2n) TO CALCULATE DISPLAY IMAGE DATA │
    │                  (t/2n + 1)                      │
    └─────────────────────────────────────────────────┘
                                    │
                                    ▼
                                   (A)                         (B)
```

# FIG. 5A

(A)                                      (B)

DISPLAY DISPLAY IMAGE DATA (t/2n + 1) — S107

EMIT LIGHT AT WAVELENGTH $\lambda 2$,
ACQUIRE ABSORPTION COEFFICIENT DISTRIBUTION (t/2n + 2) — S108

DISPLACE ABSORPTION COEFFICIENT DISTRIBUTION (t/2n + 1)
AT WAVELENGTH $\lambda 1$ — S109

CALCULATE OXYGEN SATURATION DISTRIBUTION (t/2n + 2)
FROM DISPLACED ABSORPTION COEFFICIENT DISTRIBUTION (t/2n + 1) AT WAVELENGTH $\lambda 1$
AND ABSORPTION COEFFICIENT DISTRIBUTION (t/2n + 2) AT WAVELENGTH $\lambda 1$ — S110

CALCULATE STRUCTURAL IMAGE (t/2n + 2) FROM
ABSORPTION COEFFICIENT DISTRIBUTION (t/2n + 2) — S111

TRIM OXYGEN SATURATION DISTRIBUTION (t/2n + 2) WITH
STRUCTURAL IMAGE (t/2n + 2)
TO CALCULATE DISPLAY IMAGE DATA (t/2n + 2) — S112

DISPLAY DISPLAY IMAGE DATA (t/2n + 2) — S113

END CONDITIONS SATISFIED? — S114

No → n = n + 1 — S115

Yes

END

FIG. 5B

FIG. 6

ABSORPTION COEFFICIENT

FIG. 7

FIG. 8

Europäisches
Patentamt

European
Patent Office

Office européen
des brevets

**EUROPEAN SEARCH REPORT**

Application Number

EP 18 00 0110

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2014/206960 A1 (NAGAE KENICHI [JP]) 24 July 2014 (2014-07-24) * abstract; figure 13 * * paragraphs [0016], [0061], [0063], [0073], [0102], [0108] * * the whole document * | 1-21 | INV. A61B5/00 A61B5/145 |
| X | US 2013/190589 A1 (CHEN BO [US] ET AL) 25 July 2013 (2013-07-25) * abstract * * paragraphs [0066], [0069] * | 1-21 | |
| X | WO 2016/084720 A1 (CANON KK [JP]) 2 June 2016 (2016-06-02) * abstract * * paragraphs [0007], [0009], [0049], [0051], [0079] * | 1-21 | |
| X,P | WO 2017/138408 A1 (CANON KK [JP]) 17 August 2017 (2017-08-17) * paragraphs [0063], [0067] * | 1 | |

TECHNICAL FIELDS
SEARCHED    (IPC)

A61B

The present search report has been drawn up for all claims

| Place of search | Date of completion of the search | Examiner |
|---|---|---|
| The Hague | 31 May 2018 | Furlan, Stéphane |

## ANNEX TO THE EUROPEAN SEARCH REPORT
## ON EUROPEAN PATENT APPLICATION NO.

EP 18 00 0110

This annex lists the patent family members relating to the patent documents cited in the above-mentioned European search report.
The members are as contained in the European Patent Office EDP file on
The European Patent Office is in no way liable for these particulars which are merely given for the purpose of information.

31-05-2018

| Patent document cited in search report | | | Publication date | Patent family member(s) | | | Publication date |
|---|---|---|---|---|---|---|---|
| US 2014206960 | A1 | | 24-07-2014 | BR | 112014000928 | A2 | 14-02-2017 |
| | | | | CN | 103732154 | A | 16-04-2014 |
| | | | | CN | 105796062 | A | 27-07-2016 |
| | | | | EP | 2747665 | A1 | 02-07-2014 |
| | | | | JP | 5847490 | B2 | 20-01-2016 |
| | | | | JP | 2013042996 | A | 04-03-2013 |
| | | | | RU | 2014111184 | A | 27-09-2015 |
| | | | | US | 2014206960 | A1 | 24-07-2014 |
| | | | | WO | 2013027743 | A1 | 28-02-2013 |
| US 2013190589 | A1 | | 25-07-2013 | US | 2013190589 | A1 | 25-07-2013 |
| | | | | WO | 2013112812 | A1 | 01-08-2013 |
| WO 2016084720 | A1 | | 02-06-2016 | JP | 2016101393 | A | 02-06-2016 |
| | | | | WO | 2016084720 | A1 | 02-06-2016 |
| WO 2017138408 | A1 | | 17-08-2017 | JP | 2017140092 | A | 17-08-2017 |
| | | | | WO | 2017138408 | A1 | 17-08-2017 |

EPO FORM P0459

For more details about this annex : see Official Journal of the European Patent Office, No. 12/82

**EP 3 360 467 A1**

**Patent documents cited in the description**

- JP 2016013421 A

- JP 2015142740 A